(19) Europäisches Patentamt
European Patent Office
Office européen des brevets

(11) Veröffentlichungsnummer: **0 005 828**
**B1**

(12) # EUROPÄISCHE PATENTSCHRIFT

(21) Anmeldenummer: **79101654.6**

(22) Anmeldetag: **30.05.79**

(51) Int. Cl.³: **C 07 D 403/12,**
A 61 K 31/495,
C 07 D 401/12

(54) **Neue substituierte Phenylpiperazinderivate, diese enthaltende Arzneimittel und Verfahren zu deren Herstellung.**

(30) Priorität: **06.06.78 DE 2824677**
**14.12.78 DE 2853996**

(43) Veröffentlichungstag der Anmeldung:
**12.12.79 Patentblatt 79/25**

(45) Bekanntmachung des Hinweises auf die Patenterteilung:
**11.03.81 Patentblatt 81/10**

(84) Benannte Vertragsstaaten:
**AT BE CH DE FR GB IT NL SE**

(56) Entgegenhaltungen:
**DE - A - 1 932 384**
**DE - A - 2 263 211**
**DE - A - 2 337 461**
**DE - A - 2 737 630**
**GB - A - 1 424 571**
**GB - A - 1 500 063**

(73) Patentinhaber: **HOECHST AKTIENGESELLSCHAFT**
**Zentrale Patentabteilung Postfach 80 03 20**
**D-6230 Frankfurt/Main 80 (DE)**

(72) Erfinder: **Lattrell, Rudolf, Dr.**
**Heuhohlweg 6H**
**D-6240 Königstein/Taunus (DE)**

(72) Erfinder: **Bartmann, Wilhelm, Dr.**
**Am Dachsbau 5**
**D-6232 Bad Soden am Taunus (DE)**

(72) Erfinder: **Gerhards, Hermann, Dr.**
**Kurhausstrasse 49**
**D-6238 Hofheim am Taunus (DE)**

**EP 0 005 828 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue substituierte Phenylpiperazinderivate, diese enthaltende Arzneimittel und Verfahren zu deren Herstellung

Die Erfindung betrifft neue substituierte Phenylpiperazinderivate und Verfahren zu ihrer Herstellung. Sie betrifft besonders neue Phenylpiperazinopropyloxyindolinone und -chinolinone, die wertvolle pharmakologische, insebesondere psychotrope Eigenschaften besitzen und als Arzneimittel geeignet sind.

Aus der britischen Patentschrift 1.424.571 sind Piperazinyl-2-hydroxypropyloxy-tetrahydrochinolinone mit blutdrucksenkenden Eigenschaften bekannt. Aus der deutschen Offenlegungsschrift 2.337.461 sind Phenyl-piperazinyl-propoxyindole bekannt, die ebenfalls blutdrucksenkend wirken. Darüberhinaus sind in der deutschen Offenlegungsschrift 1.932.384 2-Oxo-1,2-dihydrochinoline mit coronargefäßerweiternder und in der deutschen Offenlegungsschrift 2.263.211 Arylpiperazine mit neuroleptischer Wirkung beschrieben.

Gegenstand der Erfindung sind Phenylpiperazinderivate der Formel I

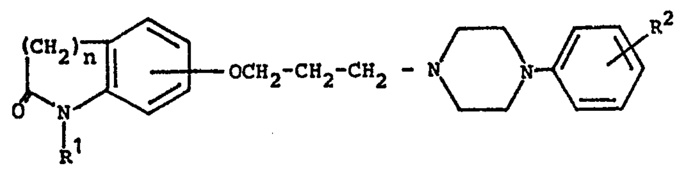

(I)

worin bedeuten

$R^1$ Wasserstoff oder eine $C_1$—$C_6$-Alkylgruppe,

$R^2$ eine oder mehrere gleiche oder verschieden Substituenten folgender Bedeutung:

$C_1$—$C_6$-Alkoxy, Halogen, Methylendioxy,

n 1 oder 2.

sowie deren physiologisch verträgliche Salze.

Als bevorzugte Substituenten kommen in Betracht:

Für $R^1$ Wasserstoff, Methyl;

für $R^2$ Methoxy, Äthoxy, Isopropoxy, n-Butoxy, Fluor, Chlor.

Gegenstand der Erfindung ist weiterhin ein Verfahren zur Herstellung von Verbindungen der Formel I, das dadurch gekennzeichnet ist, daß man

a) eine Verbindung der Formel II

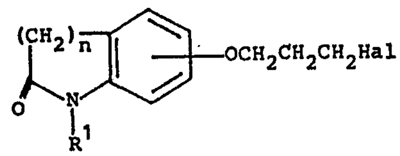

(II)

worin $R^1$ und n die obengenannte Bedeutung haben und Hal ein Halogenatom bedeutet, mit einem Phenylpiperazin der Formel III

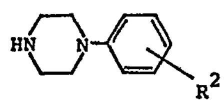

(III)

umsetzt, oder

b) eine phenolische Verbindung der Formel IV

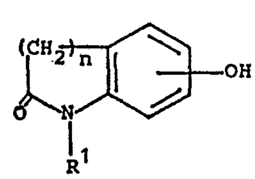

(IV)

worin $R^1$ und n die obengenannte Bedeutung haben, mit einer Verbindung der Formel V

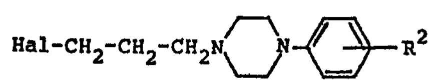

(V)

worin $R^2$ und Hal die obengenannte Bedeutung haben, umsetzt, oder

c) eine Verbindung der Formel VI

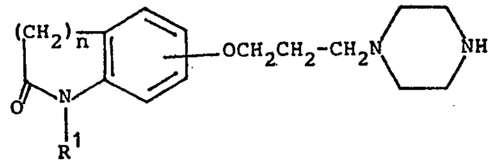

(VI)

worin $R^1$ und n die oben genannte Bedeutung haben, mit einer Verbindung der Formel VII

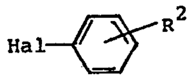

(VII)

worin $R^2$ und Hal die oben genannte Bedeutung haben, umsetzt, oder

d) eine Verbindung, der Formel VIII

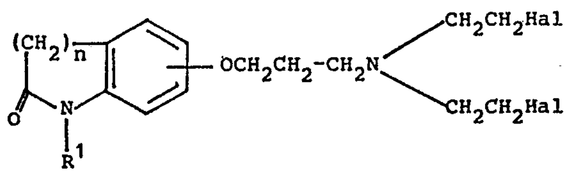

(VIII)

worin $R^1$, Hal und n die oben genannte Bedeutung haben, mit einer Verbindung der Formel IX

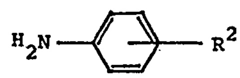

(IX)

worin $R^2$ die oben genannte Bedeutung hat, umsetzt, oder

e) eine Verbindung der Formel X

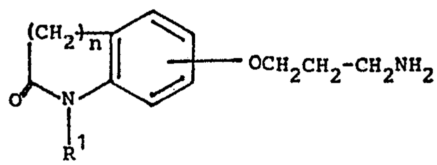

(X)

worin $R^1$ und n die oben genannte Bedeutung haben, mit einer Verbindung der Formel XI

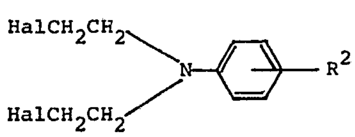

(XI)

worin $R^2$ und Hal die oben genannte Bedeutung haben, umsetzt.

Nach dem *Verfahren a* wird die Umsetzung der Verbindung der Formel II, die aus Phenolen der Formel IV und 1,3-Dihalopropanen erhalten wird, mit einem Amin der Formel III vorzugsweise in Gegenwart einer Base wie Natrium- oder Kaliumhydroxid, Natrium- oder Kaliumcarbonat, tertiären Aminen wie Triäthylamin oder Pyridin, durchgeführt, jedoch kann auch in Abwesenheit einer Base gearbeitet werden. Die Reaktion wird im allgemeinen bei Temperaturen zwischen 50° und 200°C, vorzugsweise zwischen 60° und 160°C ausgeführt. Werden Lösungsmittel verwendet, so kommen beispielsweise Äther wie Tetrahydrofuran oder Dioxan, Glykoläther wie Diglym, aromatische Kohlenwasserstoffe wie Toluol, Chlorbenzol, Alkohole wie Äthanol, Isoamylalkohol, aprotische Lösungsmittel, wie Dimethylformamid, Dimethylacetamid oder dergleichen in Frage. Das Amin der Formel III wird in mindestens äquimolaren Mengen bis zu einem fünffach-molaren Überschuß angewendet.

Nach dem *Verfahren b* wird die Umsetzung der phenolischen Verbindung der Formel VI mit Phenylpiperazinderivaten der Formel V in an sich bekannter Weise ausgeführt, wobei die vorstehend beschriebenen Reaktionsbedingungen angewendet werden. Eine bevorzugte Verfahrensvariante besteht darin, daß man zunächst die phenolische Verbindung der Formel IV mittels eines Alkalialkoholates oder eines Alkalihydrids in das entsprechende Alkalisalz überführt.

Nach dem *Verfahren c* werden die monosubstituierten Piperazinderivate der Formel VI, die ihrerseits nach de Verfahrensvariante b) aus Verbindungen der allgemeinen Formel II und Piperazin erhalten werden, mit Verbindungen der Formel VII kondensiert. Die Umsetzung wird vorzugsweise in einem polaren Lösungsmittel, z.B. Alkoholen wie Isoamylalkohol, Äthern wie Diglym oder aprotischen Lösungsmittel wie Dimethylformamid, bei Temperaturen zwischen 60 und 200°C in Gegenwart eines Akzeptors für die im Verlauf der Reaktion gebildete Halogenwasserstoffsäure, beispielsweise Kaliumcarbonat, ausgeführt.

Nach dem *Verfahren d* wird die Kondensation der Verbindungen VIII mit Anilinderivaten der Formel IX in einem Lösungsmittel, wie sie vorstehend genannt wurden, bei einer Temperatur zwischen 60 und 160°C, vorzugsweise in Gegenwart eines Halogenwasserstoffakzeptors, wie z.B. Kaliumcarbonat oder Pyridin, ausgeführt.

Nach dem *Verfahren e* werden Verbindungen der Formel X, die ihrerseits in an sich bekannter Weise aus Verbindungen der Formel II erhalten werden, mit N-(Bis-Halogenäthyl)-anilinen, kondensiert, wobei die für die Verfahren c) und d) beschriebenen Reaktionsbedingungen angewendet werden.

Die Verbindungen der allgemeinen Formel I werden in freier Form oder als Salze isoliert, je nach den angewandten Reaktionsbedingungen. Die freien Basen können nach Reaktion mit anorganischen oder organischen Säuren in ihre pharmakologisch verträglichen Salze übergeführt werden. Solche Säuren sind z.B. Salzsäure, Schwefelsäure, Phosphorsäure, aliphatische, alicyclische, araliphatische, aromatische oder heterocyclische Carbonsäuren oder Sulfonsäuren wie Essigsäure, Weinsäure, Milchsäure, Maleinsäure, Fumarsäure, Zitronensäure, Oxalsäure, Methansulfonsäure, Hydroxyäthansulfonsäure oder synthetische Harze, die saure Gruppen enthalten.

Die erfindungsgemäßen Verbindungen sind neue Verbindungen die als pharmakologische Mittel geeignet sind. Sie zeigen insbesondere neuroleptische Wirksamkeit. So antagonisieren die Verbindungen dosisabhängig die Amphetamin-Aggregations-Toxizität bei Mäusen ($ED_{50}$-Werte zwischen 0,5 und 20 mg/kg). In diesem Test erhalten Gruppen von je 10 Mäusen, die auf engem Raum zusammensitzen (ca. 25 $cm^2$/Maus) eine Stunde nach Gabe der Verbindung 20 mg/kg D-Amphetamin in 0,2% wässriger Lösung s.c. injiziert. Es wird die Dosis der Verbindung ermittelt, die 50% der Tiere vor dem Tod durch die Amphetaminvergiftung schützt. Desweiteren hemmen die Verbindungen die Bindung von Tritium-markiertem Spiroperidol an Zellmembranbestandteile aus Homogenat von Dopamin-reichen Hirnarealen (corpus striatum) von Ratte und Kalb ($^3$H-Spiroperidol-Bindungstest J.Z. Fields et al, Brain Res. *136,* 578 (1977). Die Konzentrationen der Verbindungen, die für eine 50% Hemmung erforderlich sind ($IC_{50}$), liegen zwischen $2,6 \times 10^{-6}$ und $1,0 \times 10^{-7}$ Mol/1.

Einige der Verbindungen haben keine oder nur eine schwache kataleptogene Wirkung, d.h. sie bewirken nur in hohen Dosen (> 40 mg/kg) eine kataleptische Starre bei Ratten.

Die neuen Verbindungen können entweder allein oder mit physiologisch verträglichen Hilfs- oder Trägerstoffen vermischt angewandt werden. Sie können oral, parenteral oder intravenös verabreicht werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. als inerte Träger können z.B. Magnesiumcarbonat, Milchzucker oder Maisstärke under Zusatz anderer Stoffe wie z.B. Magnesiumstearat verwendet werden. Dabei kann die Zubereitung sowohl als Trocken- oder Feuchtgranulat erfolgen. Als ölige Trägerstoffe oder Lösungsmittel kommen besonders pflanzliche oder tierische Öle in Betrachte wie z.B. Sonnenblumenöl oder Lebertran.

Als Lösungsmittel der entsprechenden physiologisch verträglichen Salze der aktiven Verbindungen für eine intravenöse Applikation kommen z.B. in Frage: Wasser, physiologische Kochsalzlösung oder Alkohole wie z.B. Äthanol, Propandiol oder Glycerin, daneben auch Zuckerlösungen wie z.B. Glukose- oder Mannitlösungen, oder auch eine Mischung aus den verschiedenen genannten Lösungsmitteln.

Die erfindungsgemäßen Verbindungen und ihre pharmakologisch verträglichen Salze sind innerhalb eines breiten Dosierungsbereiches wirksam. Die Höhe der verabreichten Dosis ist selbstverständlich abhängig von der Art der gewünschten Behandlung, von der Verabreichungsweise und vom Zustand, vom Typ und von der Größe des zu behandelnden Säugetieres. Bei oraler Verabreichung werden befriedigende Ergebnisse mit Dosen von 0,1—100 mg an aktiver Substanz pro kg Tierkörpergewicht erzielt, beim Menschen variiert die tägliche Dosis zwischen 20—800 mg an aktiver Substanz pro Mensch, vorzugsweise zwischen 50—500 mg, wobei Einzeldosen von 20—200 mg, vorzugsweise ein bis dreimal täglich gegeben werden können. Für intravenöse oder intramuskuläre Anwendung beträgt die Dosis 5—300 mg, vorzugsweise 10—200 mg täglich.

Beispiel 1
5-[3-{4-(4-Chlorphenyl)-1-piperazinyl}-propoxy]-1-methyl-2-indolinon

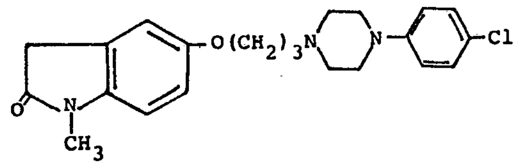

*Stufe 1:* Darstellung von 5-(3-Brompropoxy)-1-methyl-2-indolinon:

Eine Gemisch aus 11,9 g 5-Hydroxy-1-methyl-2-indolinon, 29,2 g 1,3-Dibrompropan, 11 g Kaliumcarbonat, 1 g Kaliumjodid und 75 ml Äthylmethylketon werden 20 Stunden unter Rückfluß gekocht. Die Mischung wird mit 300 ml Methylendichlorid verdünnt, mit Wasser gewaschen und das Lösungsmittel im Vakuum entfernt.

Das erhaltene Rohprodukt wird über Kieselgel (Desaktiviert mit 10% Wasser) mit Äthyleacetat: Cyclohexan (1:1) filtriert. Nach einem Verlauf wird ein Öl eluiert, das dünnschichtchromatographisch einheitlich ist und direkt weiter verwendet wird.

*Stufe 2:*

2,8 g (0,01 Mol) 5-(3-Brompropoxy)-1-methyl-2-indolinon, 2 g (0,01 Mol) 1-(4-Chlorphenyl)-piperazin, 2,6 g Kaliumcarbonat und 20 ml Toluol werden 12 Stunden unter Rückfluß gerührt. Die erkaltete Mischung wird mit 100 ml Methylendichlorid verdünnt, mit Wasser gewaschen und das Lösungsmittel im Vakuum entfernt.

Der amorphe Rückstand der organischen Phase wird in 100 ml Aceton gelöst. Nach Zugabe eines Überschusses an äthanolischer Salzsäure bildet sich ein kristalliner Niederschlag, der abgesaugt und mit Aceton gewaschen wird. Ausbeute 4,1 g (87% d.Th.) Dihydrochlorid der Titelverbindung vom Schmp. 170—173°C (Zersetzung).

In Analogie zu Beispiel 1 werden aus dem 5-(3-Brompropoxy)-1-methyl-2-indolinon die folgenden Derivate hergestellt.

Beispiel 2
5-[3-{4-(2-Methoxyphenyl)-1-piperazinyl}-propoxy]1-methyl-2-indolinon
Ausbeute: 92% d.Th. Dihydrochlorid Schmp. 210—211°C Zersetzung.

Beispiel 3
5-[3-{4-(3-Chlorphenyl)-1-piperazinyl}-propoxy]-1-methyl-2-indolinon
Ausbeute: 88% d.Th., Dihydrochlorid Schmp. 205°C Zersetzung.
Freie Base: Schmp. 107°C.

Beispiel 4
6-[3-{4-(2-Methoxyphenyl)-1-piperazinyl}-propoxy]-1,2,3,4-tetrahydrochinolin-2-on

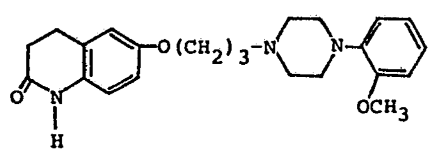

*Stufe 1:* Darstellung von 6-(3-Brompropoxy)-1,2,3,4-tetrahydrochinolinon-2:

Ein Gemisch aus 11,9 g 6-Hydroxy-1,2,3,4-tetrahydrochinolin-2-on, 22,4 g 1,3-Dibrompropan, 11 g Kaliumcarbonat, 1 g Kaliumjodid und 75 ml Äthylmethylketon werden 20 Stunden unter Rückfluß gekocht. Die Mischung wird mit 300 ml Methylendichlorid verdünnt, mit Wasser gewaschen und das Lösungsmittel im Vakuum entfernt. Der Rückstand wird mit Äther verrieben, die Kristalle abgesaugt und mit Äther gewaschen.
Ausbeute: 8,6 g, Schmp. 113—114°C.

*Stufe 2*

2,8 g (0,01 Mol) 6-(3-Brompropoxy)-1,2,3,4-tetrahydrochinolinon-2, 1,92 g (0,01 Mol) 1-(2-Methoxyphenyl)-piperazin, 4,2 g Kaliumcarbonat und 25 ml Toluol werden 5 Stunden unter Rückfluß gekocht. Die Mischung wird mit Methylendichlorid verdünnt, mit Wasser gewaschen und das Lösungsmittel im Vakuum entfernt. Der amorphe Rückstand wird in Aceton gelöst, ein Überschuß äthanolischer Salzsäure zugegeben, der gebildete Niederschlag abgesaugt und mit Aceton gewaschen.
Ausbeute: 4 g (85% d.Th.), Schmp. 190—192°C.
Dihydrochlorid.

Beispiel 5

6-[3-{4-(4-Chlorphenyl)-1-piperazinyl}-propoxy]-1,2,3,4-tetrahydrochinolin-2-on

In Analogie zu Beispiel 4 aus 6-(3-Brompropoxy)-1,2,3,4-tetrahydrochinolin-2-on und 1-(4-Chlorphenyll)-piperazin hergestellt. Ausbeute 80% d.Th. Base Schmp. 205—206°C Dihydrochlorid: Schmp. 212—214°C.

**Patentansprüche für die Vertragstaaten: BE, CH, DE, FR, GB, IT, NL, SE**

1. Phenylpiperazinderivate der Formel I

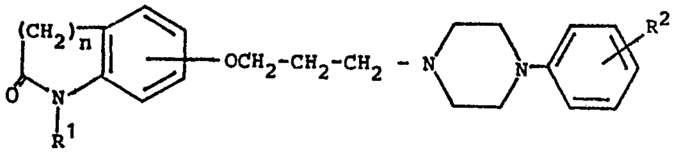

(I)

worin bedeuten

$R^1$ Wasserstoff oder eine $C_1$—$C_6$-Alklygruppe,

$R^2$ eine oder mehrere gleiche oder verschiedene Substituenten folgender Bedeutung: $C_1$—$C_6$-Alkoxy, Halogen, Methylendioxy,

n 1 oder 2

sowie deren physiologisch verträglichen Salze.

2. Verfahren zur Hellstellung von Verbindungen der Formel I, dadurch gekennzeichnet, daß man

a) eine verbindung der Formel II

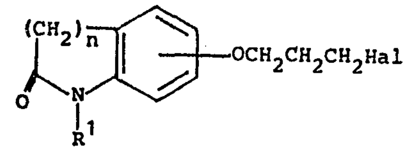

(II)

worin $R^1$ und n die obengennante Bedeutung haben und Hal ein Halogenatom bedeutet, mit einem Phenylpiperazin der Formel III

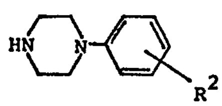

(III)

umsetzt, oder.

b) eine phenolische Verbindung der Formel IV

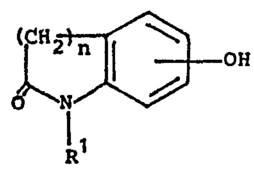

(IV)

worin $R^1$ und n die obgenannte Bedeutung haben, mit einer Verbindung der Formel V

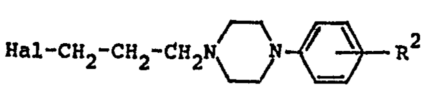

(V)

worin $R^2$ und Hal die obengenannte Bedeutung haben, umsetzt, oder

c) eine Verbindung der Formel VI

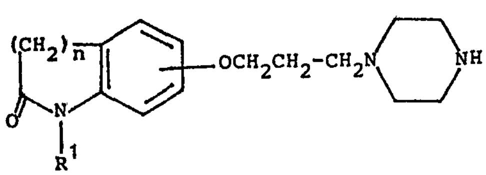

(VI)

worin $R^1$ und n die oben genannte Bedeutung haben, mit einer Verbindung der Formel VII

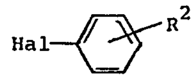

(VII)

worin $R^2$ und Hal die oben genannte Bedeutung haben, umsetzt, oder

d) eine Verbindung der Formel VIII

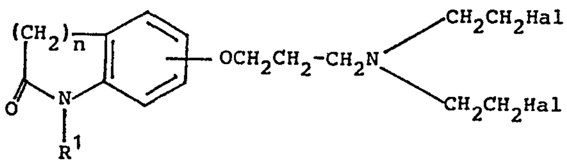

(VIII)

worin $R^1$, Hal und n die oben genannte Bedeutung haben, mit einer Verbindung der Formel IX

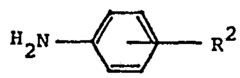

(IX)

worin $R^2$ die oben genannte Bedeutung hat, umsetzt, oder

e) eine Verbindung der Formel X

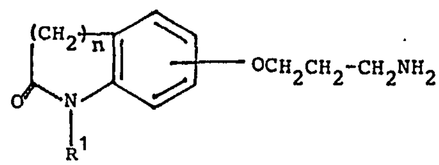

(X)

worin $R^1$ und n die oben genannte Bedeutung haben, mit einer Verbindung der Formel XI

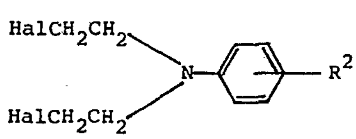

(XI)

worin $R^2$ und Hal die oben genannte Bedeutung haben, umsetzt.

3. Verfahren zur Herstellung von Arzneimitteln, dadurch gekennzeichnet, daß man Verbindungen der Formel I von Anspruch 1 gegebenenfalls zusammen mit üblichen pharmazeutischen Trägern und/oder Stabilisatoren in eine für therapeutische Zwecke geeignete Darreichungsform bringt.

4. Arzneimittel, gekennzeichnet durch einen Gehalt an einer Verbindung nach Anspruch 1 oder bestehend aus einer solchen Verbindung.

**Claims for the contracting states: BE, CH, DE, FR, GB, IT, NL, SE**

1. Phenylpiperazine derivatives of the formula I

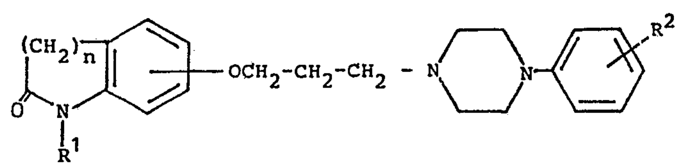

(I)

in which

$R^1$ denotes hydrogen or $C_1$—$C_6$alkyl,

$R^2$ denotes one or several identical or different substituents selected from $C_1$—$C_6$alkoxy, halogen and methylene-dioxy,

and

n is 1 or 2,

and the physiologically acceptable salts thereof.

2. Process for the manufacture of a compound of the formula I as defined in claim 1 which comprises

a) reacting a compound of the formula II

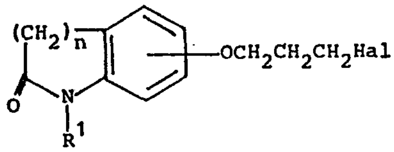

(II)

in which $R^1$ and n are as defined in claim 1 and Hal denotes a halogen atom, with a phenylpiperazine of the formula III in which $R^2$ is as defined in Claim 1,

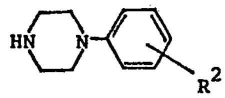

(III)

b) reacting a phenolic compound of the formula IV

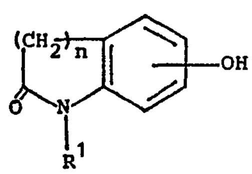

(IV)

in which R1 and n are as defined in claim 1; with a compound of the formula V

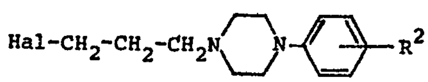

(V)

in which $R^2$ is as defined in claim 1 and Hal denotes a halogen atom;

c) reacting a compound of the formula VI

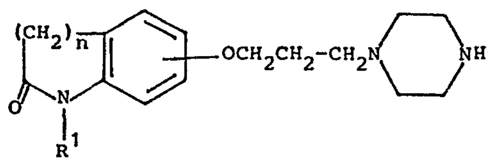

(VI)

in which $R^1$ and n are as defined in claim 1, with a compound of the formula VII

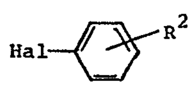

(VII)

in which $R^2$ and Hal are as defined above,

d) reacting a compound of the formula VIII

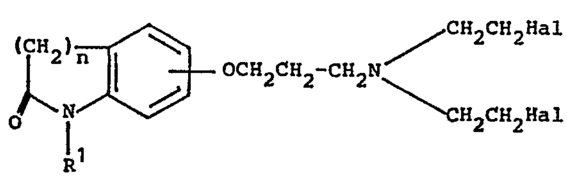

(VIII)

in which $R^1$, Hal and n are as defined above, with a compound of formula IX

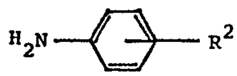

(IX)

in which $R^2$ is as defined in claim 1;

e) reacting a compound of the formula X

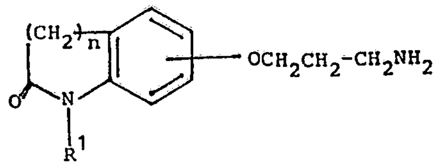

(X)

in which $R^1$ and n are as defined in claim 1, with a compound of the formula XI

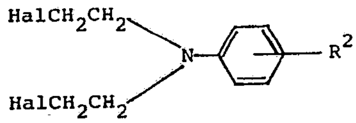

(XI)

in which $R^2$ and Hal are as defined above.

3. Process for the manufacture of a medicament, which comprises bringing a compound as claimed in claim 1 in a suitable form of administration for therapeutic purposes, if desired together with the usual pharmaceutical carriers and/or stabilizers.

4. Medicament containing an effective amount of a compound as claimed in claim 1 or consisting of such a compound.

**Revendications pour les Etats contractants: BE, CH, DE, FR, GB, IT, NL, SE**

1. Dérivés de la phénylpipérazine de formule I

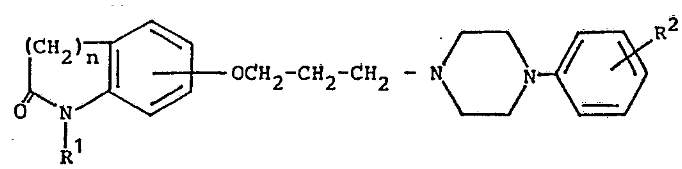

(I)

dans laquelle

$R^1$ désigne l'hydrogène ou un groupe alcoyle en $C_1$ à $C_6$

$R^2$ désigne un ou plusieurs substituants choisis parmi:

alcoxy en $C_1$ à $C_6$, halogènes et méthylènedioxy,

n est égal à 1 ou 2,

ainsi que leurs sels physiologiquement acceptables.

2. Procédé de préparation de composés répondant à la formule I, caractérisé en ce que

a) on fait réagir un composé de formule II

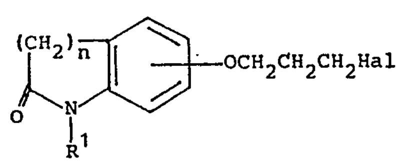

(II)

dans laquelle $R^1$ et n ont les significations ci-dessus indiquées et Hal désigne un atome d'halogene, avec une phénylpipérazine de formule III

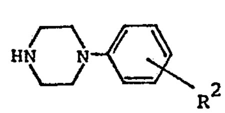

(III)

ou

b) on fait réagir un composé phénolique de formule IV

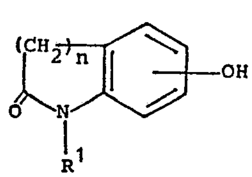

(IV)

dans laquelle R¹ et n ont la signification ci-dessus indiquée, avec un composé de formule V

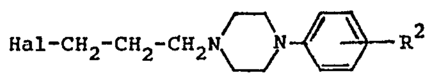
(V)

dans laquelle R² et Hal ont les signifìcations ci-dessus indiquées
ou

c) on fait réagir un composé de formule VI

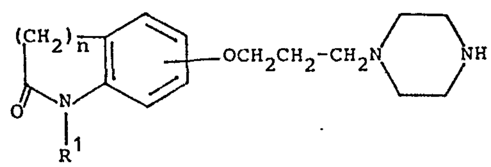
(VI)

dans laquelle R¹ et n ont la signification ci-dessus indiquée, avec un composé de formule VII

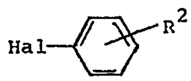
(VII)

dans laquelle R² et Hal ont les significations ci-dessus indiquées, ou

d) on fait réagir un composé de formule VIII

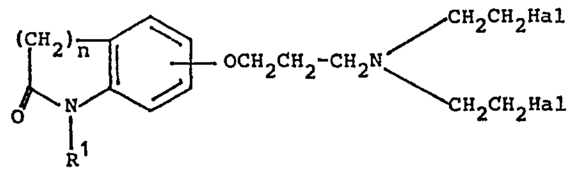
(VIII)

dans laquelle R¹, Hal et n ont les significations indiquées ci-dessus, avec un composée de formule IX

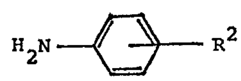
(IX)

dans laquelle R² a la signification indiquée ci-dessus, ou

e) on fait réagir un composé de formule X

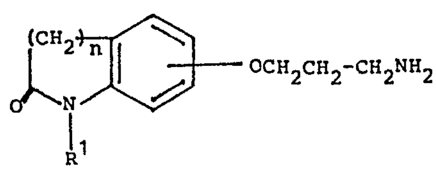
(X)

dans laquelle R¹ et n ont les significations ci-dessus indiquées, avec un composé de formule XI

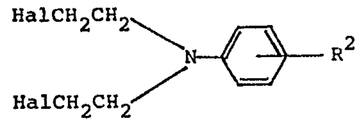
(XI)

dans laquelle R² et Hal ont les significations ci-dessus indiquées.

3. Procédé de préparation de médicaments, caractérisé en ce qu'on amène des composés répondant à la formule I de la revendication 1, en même temps que le cas échéant, des véhicules et/ou stabilisants pharmaceutiques habituels, sous une forme d'administration appropriée aux buts thérapeutiques poursuivis.

4. Médicament, caractérisé en ce qu'il contient un composé suivant la revendication 1, ou en ce qu'il est constitué d'un tel composé.

**Patentanspruche für den Vertragsstaat: AT**

1. Verfahren zur Herstellung von Verbindungen der Formel I,

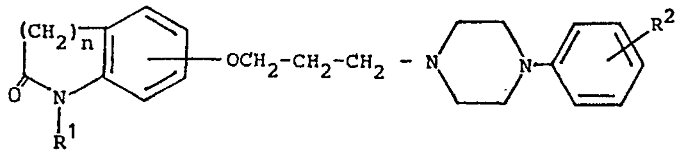

(I)

worin bedeuten

$R^1$ Wasserstoff oder eine $C_1$—$C_8$-Alkylgruppe,

$R^2$ eine oder mehrere gleiche oder verschiedene Substituenten folgender Bedeutung: $C_1$—$C_6$-Alkoxy, Halogen, Methylendioxy,

n 1 oder 2,

sowie deren physiologisch verträglichen Salze dadurch gekennzeichnet, daß man

a) eine Verbindung der Formel II

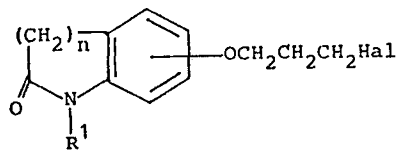

(II)

worin $R^1$ und n die obengenannte Bedeutung haben und Hal ein Halogenatom bedeutet, mit einem Phenylpiperazin der Formel III

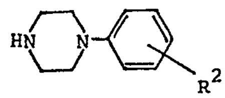

(III)

umsetzt, oder.

b) eine phenolische Verbindung der Formel IV

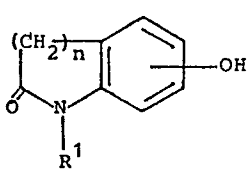

(IV)

worin $R^1$ und n die obengenannte Bedeutung haben, mit einer Verbindung der Formel V

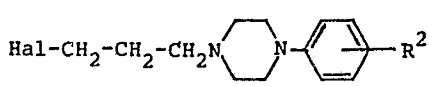

(V)

worin $R^2$ und Hal die obengenannte Bedeutung haben, umsetzt, oder

c) eine Verbindung der Formel VI

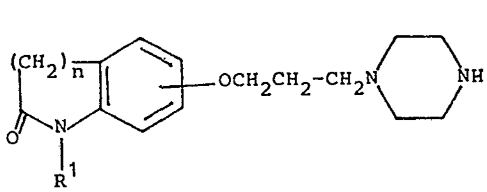

(VI)

worin $R^1$ und n die oben genannte Bedeutung haben, mit einer Verbindung der Formel VII

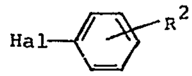

(VIII)

worin $R^2$ und Hal die oben genannte Bedeutung haben, umsetzt, oder

d) eine Verbindung der Formel VIII

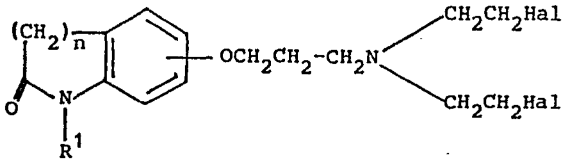

(VIII)

worin $R^1$ Hal und n die oben genannte Bedeutung haben, mit einer Verbindung der Formel IX

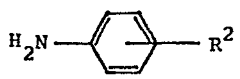

(IX)

worin $R^2$ die oben genannte Bedeutung hat, umsetzt, oder

e) eine Verbindung der Formel X

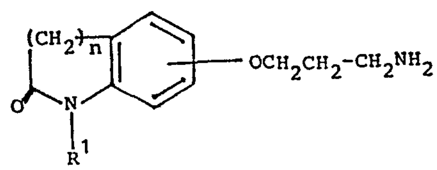

(X)

worin $R^1$ und n die oben genannte Bedeutung haben, mit einer Verbindung der Formel XI

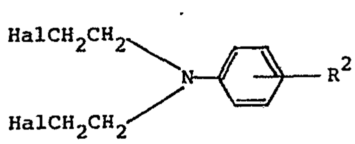

(XI)

worin $R^2$ und Hal die oben genannte Bedeutung haben, umsetzt.

2. Verfahren zur Herstellung von Arzneimitteln, dadurch gekennzeichnet, daß man Verbindungen der Formel I von Anspruch 1 gegebenenfalls zusammen mit üblichen pharmazeutischen Trägern und/oder Stabilisatoren in eine für therapeutische Zwecke geeignete Darreichungsform bringt.

**Claims for the contracting state: AT**

1. Process for the manufacture of compounds of the formula I

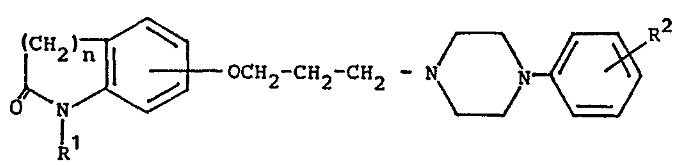

(I)

in which

R1 denotes hydrogen or $C_1$—$C_6$-alkyl,

$R^2$ denotes one or several identical or different substituents selected from $C_1$—$C_6$alkoxy, halogen and methylene-dioxy,

and

n is 1 or 2,

and the physiologically acceptable salts thereof, which comprises

a) reacting a compound of the formula II

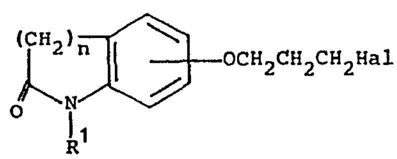

(II)

in which $R^1$ and n are as defined as above and Hal denotes a halogen atom, with a phenylpiperazine of the formula III in which $R^2$ is as defined above,

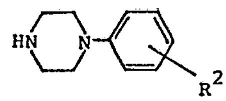

(III)

b) reacting a phenolic compound of the formula IV

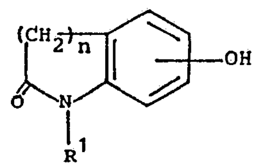

(IV)

in which $R^1$ and n are as defined above, with a compound of the formula V

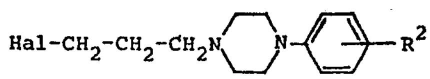

(V)

in which $R^2$ is as defined above and Hal denotes a halogen atom;

c) reacting a compound of the formula VI

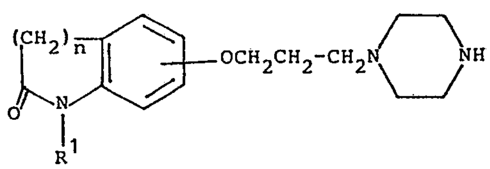

(VI)

in which $R^1$ and n are as defined above, with a compound of the formula VII

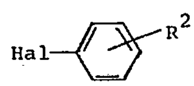

(VII)

in which $R^2$ and Hal are as defined above,

d) reacting a compound of the formula VIII

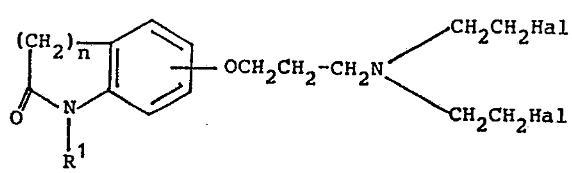

(VIII)

in which $R^1$, Hal and n are as defined above, with a compound of the formula IX

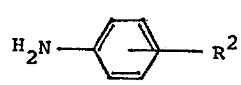

(IX)

in which $R^2$ is as defined above

e) reacting a compound of the formula X

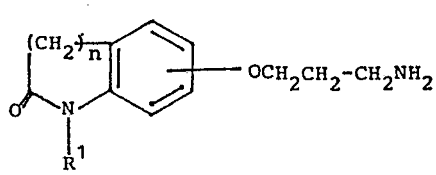

(X)

in which $R^1$ and n are as defined above, with a compound of the formula XI

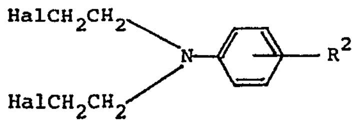

(XI)

in which $R^2$ and Hal are as defined above.

2. Process for the manufacture of a medicament, which comprises bringing a compound of formula I of claim 1 in a suitable form of administration for therapeutic purposes, if desired together with the usual pharmaceutical carriers and/or stabilizers.

**Revendications pour l'Etat contractant: AT**

1. Procédé de préparation de composés répondant à la formula I

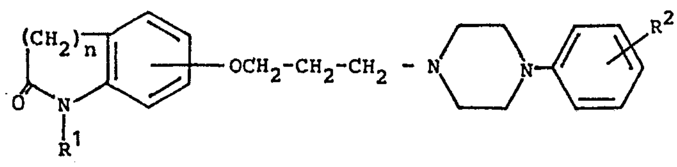

(I)

dans laquelle
$R^1$ désigne l'hydrogène ou un groupe alcoyle en $C_1$ à $C_6$
$R^2$ désigne un ou plusieurs substituants choisis parmi:
alcoxy en $C_1$ à $C_6$, halogènes et méthylènedioxy,
n est égal à 1 ou 2,
ainsi que leurs sels physiologiquement acceptables, caractérisé en ce que

a) on fait réagir un composé de formule II

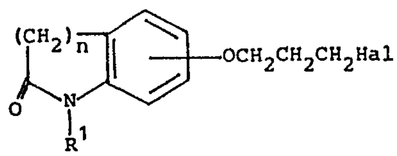

(II)

dans laquelle $R^1$ et n ont les significations ci-dessus indiquées et Hal désigne un atome d'halogène, avec une phénylpipérazine de formule III

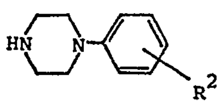

(III)

ou

b) on fait réagir un composé phénolique de formule IV

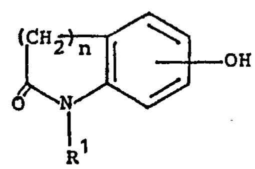

(IV)

dans laquelle $R^1$ et n ont la signification ci-dessus indiquée, avec un composé de formule V

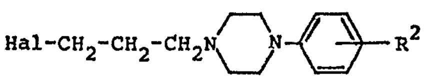

(V)

dans laquelle $R^2$ et Hal ont les significations ci-dessus indiquées
ou

c) on fait réagir un composé de formule VI

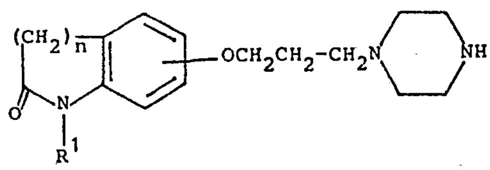

(VI)

dans laquelle $R^1$ et n ont la signification ci-dessus indiqueé, avec un composé de formule VII

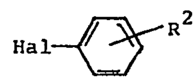

(VIII)

dans laquelle $R^2$ et Hal ont les significations ci-dessus indiquées, ou

d) on fait réagir un composé de formule VIII

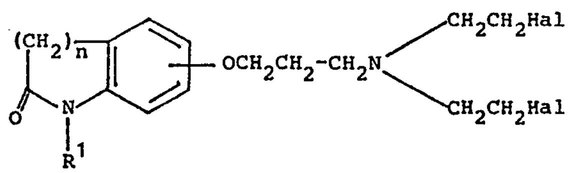

(VIII)

dans laquelle $R^1$, Hal et n ont les significations indiquées ci-dessus, avec un composé de formule IX

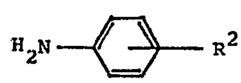

(IX)

dans laquelle $R^2$ a la signification indiquée ci-dessus, ou

e) on fait réagir un composé de formule X

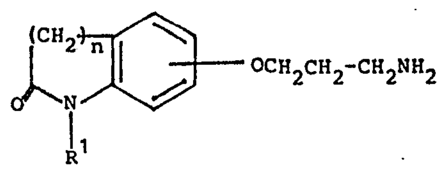

(X)

dans laquelle $R^1$ et n ont les significations ci-dessus indiquées, avec un composé de formule XI

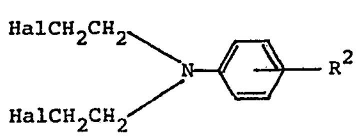

(XI)

dans laquelle $R^2$ et Hal ont les significations ci-dessus indiquées.

2. Procédé de préparation de médicaments, caractérisé en ce qu'on amène des composé répondant à la formule I de la revendication 1, en même temps que le cas échéant, des véhicules et/ou stabilisants pharmaceutiques habituels, sous une forme d'administration appropriée aux buts thérapeutiques poursuivis.